# EUROPÄISCHE PATENTANMELDUNG

(11) **EP 1 132 460 A2**
(43) Veröffentlichungstag der Anmeldung: **12.09.2001**
(21) Anmeldenummer: 01105150.5
(22) Anmeldetag: 02.03.2001
(51) Int. Cl.: C12M 3/04

(54) **Bioreaktor und Verfahren zum Züchten dendritischer Zellen**

(30) Priorität: 06.03.2000 DE 10010950
(71) Anmelder: Sefar AG, 8803 Rüschlikon (CH)
(72) Erfinder: Mayer, Christine, Mag. rer. nat., 70569 Stuttgart (DE); Graeve, Thomas, Dr., 70372 Stuttgart (DE)
(74) Vertreter: Wunderlich, Rainer, Dipl.-Ing.

(57) **Zusammenfassung**

Die Erfindung betrifft die Anordnung eines Zellträgers zwischen zwei Membranabschnitten, wobei sich eine beutelartige Struktur ergibt. Die Erfindung betrifft weiterhin Verfahren zum Züchten dendritischer Zellen, bei welchen insbesondere die erfindungsgemäßen Bioreaktoren zum Einsatz kommen.

## Beschreibung

Die Erfindung betrifft einen Bioreaktor zur Kultivierung organischen Materials mittels eines Nährmediums und ein Verfahren zum Züchten dendritischer Zellen.

Die Kultivierung organischen Materials, vor allem menschlicher oder tierischer Zellen gewinnt in der medizinischen Diagnostik und Therapie sowie in der Pharmakologie zunehmend an Bedeutung. Besonders die Verwendung dendritischer Zellen wird in der Zukunft eine wichtige Rolle in der Immuno-Therapie spielen. Das Prinzip dieser Verwendung besteht im Wesentlichen darin, dendritische Zellen an ihrer Oberfläche zu modifizieren, so dass die Dendriten Marker für Tumorzellen erhalten. Die Dendriten werden dann zur Aktivierung des Immunsystems eingesetzt. Allerdings ist die Züchtung dendritischer Zellen derzeit fast ausschließlich im Labormaßstab möglich und mit großem Aufwand verbunden.

Der Erfindung liegt die **Aufgabe** zugrunde, eine Vorrichtung und ein Verfahren zu schaffen, mit welchen die Kultivierung von Zellen verbessert wird, so dass insbesondere dendritische Zellen einfach und zuverlässig gezüchtet werden können.

Diese Aufgabe wird mit den Merkmalen der Ansprüche 1, 11 und 15 gelöst.

Ein erfindungsgemäßer Bioreaktor ist dadurch gekennzeichnet, dass ein Trägerelement zum Tragen des organischen Materials vorgesehen ist, wobei das Trägerelement für das Nährmedium zumindest teilweise durchlässig ist, und dass das Trägerelement zwischen zwei Abschnitten mindestens eines flächigen Elementes angeordnet und umschlossen ist, wobei eine beutelartige Struktur gebildet ist. Eine solche beutelartige Struktur lässt sich besonders einfach handhaben, beispielsweise zur Platzierung in einem Brutschrank, und sie lässt sich sehr kostengünstig herstellen. Dies ist insbesondere bei Verwendung als hygienischer Einmalartikel besonders wichtig. Ist die beutelartige Struktur nach außen durch eine Folie begrenzt, so ist der Träger in dem Beutel nach außen vollständig isoliert oder allenfalls gasdurchlässig. Verwirklicht man den Beutel alternativ durch Membranen, welche für ein Nährmedium durchlässig sind, so kann der Beutel in einem Nährmedium angeordnet und von diesem gegebenenfalls durchströmt werden. Bei Undurchlässigkeit der Membran für das gezüchtete organische Material kann somit eine gute bzw. stets frische Nährstoffversorgung sichergestellt werden. Ein besonders guter Nährstoffdurchsatz wird durch die erfindungsgemäße Verwendung eines Gewebes erreicht. Die Zwischenräume können dabei so ausgebildet sein, dass sich eine gezielte Diffusorwirkung ergibt.

Grundsätzlich kann der beutelförmige Bioreaktor beim Verschweißen befüllt werden. Es ist aber vorteilhaft, wenn eine Zuführung und eine Abführung für das Nährstoffmedium vorgesehen sind. Dann kann der Beutel aus einer undurchlässigen Folie gefertigt sein, und es kann dennoch ein strömendes Nährmedium verwendet werden. Die Zuführung und die Abführung können ebenfalls zum Beimpfen des Ansatzes verwendet werden.

Die Membran, die Folie oder das Gewebe können durch Stützelemente verstärkt sein. Der Beutel wird auf diese Weise stabilisiert, was auch im Hinblick auf einen möglichst konstanten Aufnahmeraum innerhalb des Beutels von Vorteil ist.

Eine Stabilisierung kann vorzugsweise dadurch erfolgen, dass die Membran oder die Folie ein Stützgewebe aufweist.

In einer bevorzugten Ausführungsform eines Bioreaktors ist das Trägerelement zwischen dem flächigen Element eben angeordnet. Diese flächige Anordnung ist besonders einfach und unkompliziert.

Es kann aber auch vorteilhaft sein, dass das Trägerelement zwischen dem flächigen Element wellenförmig oder wabenartig angeordnet ist. Hierdurch vergrößert sich die Oberfläche des Trägerelementes, was im Hinblick auf die Ausbeute gezüchteter Zellen Vorteile mit sich bringt.

Es kann auch vorteilhaft sein, dass das Trägerelement auf Platten angeordnet ist. Dies stabilisiert das Trägerelement, was angesichts der Strömung des Nährmediums nützlich sein kann und was ferner eine Entnahme des Trägerelementes aus dem Bioreaktor erleichtert.

Das Trägerelement kann verschiedene Gestalt haben. Es kann plissiert, gekreppt, gerollt, gefaltet oder geschichtet sein; ferner kann er aus einem dreidimensional gewobenen Material bestehen. Die verschiedenen Gestalten des Trägerelementes können so der jeweilig durchzuführenden Kultivierung angepasst werden.

Gemäß einem zweiten Aspekt der Erfindung wird die Aufgabe dadurch gelöst, dass ein Trägerelement zum Tragen des organischen Materials vorgesehen ist, wobei das Trägerelement für das Nährmedium zumindest teilweise durchlässig ist, dass das Trägerelement von einem Gehäuse umschlossen ist, welches ein Stützgerüst umfasst, und dass an dem Stützgerüst ein flächiges Element, insbesondere eine Membran, eine Folie oder ein Gewebe angeordnet ist. Durch das Stützgerüst wird ein definierter Aufnahmeraum in den Bioreaktor sichergestellt, so dass die Kultivierung des organischen Materials in einem weitgehend konstanten Zustand durchführbar ist. Das Stützgerüst kann aus verschiedensten starren Materialien aufgebaut sein.

Eine bevorzugte Ausführungsform besteht jedoch darin, dass das Stützgerüst als ein ein- oder mehrteiliges Spritzgussteil ausgebildet ist. Das Stützgerüst kann so in kostengünstiger Weise in großen Stückzahlen hergestellt werden. Dies ist für den Einsatz als ein Einmalartikel im medizinischen Bereich von wesentlicher Bedeutung.

Die Erfindung ist weiter dadurch vorteilhaft ausgebildet, dass mindestens eine Zuführung und mindestens eine Abführung für das Medium an dem Stützgerüst vorgesehen sind. Die Zuführungen sind Rohr- oder Leitungsstutzen, welche in einfache Weise einstückig mit dem Stützgerüst ausgebildet werden.

Gemäß einer anderen Weiterbildung der Erfindung ist vorgesehen, dass das Stützgerüst zumindest zwei Teile umfasst, dass an dem ersten Teil das flächige Element, insbesondere die Membran, die Folie oder das Gewebe, angebracht ist, und dass an dem zweiten Teil das Trägergerüst angebracht ist.

So kann das Stützgerüst beispielsweise als ein zylindrischer oder sonstiger Volumenkörper mit relativ großen Öffnungen ausgebildet werden, an welchem die Membran, die Folie oder das Gewebe in fertigungstechnisch einfacher Weise angebracht, insbesondere angeklebt oder festgeschweißt wird. Das zumindest eine weitere Teil kann dann als eine Verschlussplatte ausgebildet werden, welche zum dichten Verschließen des mit dem flächigen Element versehenen Teiles daran an bringbar ist. In bevorzugter Weise kann an dem zweiten Teil das Trägerelement fest angebracht sein, so dass eine einfache und zugleich lagegenaue Anordnung des Trägerelementes innerhalb des Bioreaktors erzielbar ist.

Bei den vorausgehend beschriebenen Ausgestaltungen der Erfindung wird ein Trägerelement eingesetzt, welches zur Aufnahme und zum Halten des organischen Materials geeignet ist. Dies können insbesondere verschiedene Membrane oder Gewebe sein, deren Oberfläche zur Aufnahme des Zellenmateriales modifiziert sind. Zur Oberflächenbehandlung des Trägerelementes können verschiedene chemische oder physikalische Beschichtungsverfahren, beispielsweise eine sogenannte Plasmamodifizierung, eingesetzt werden.

Die Erfindung besteht ferner in einem Verfahren zum Züchten insbesondere dendritischer Zellen, bei dem ein Trägerelement auf einen Membran- oder Folienabschnitt mindestens eines flächigen Elementes, insbesondere einer Membran, einer Folie oder eines Gewebes, gelegt wird, das flächige Element zu einem Beutel zusammengeschweißt wird, organisches Material, insbesondere Monozyten und Nährmedium in den Beutel eingebracht werden, der Beutel inkubiert wird, wobei die Zellen heranwachsen, und die Zellen vom Trägerelement gelöst und geerntet werden. Mit diesem Verfahren gelingt es, dass sich beispielsweise Monozyten fest an das Trägerelement anlagern, während sich die dendritischen Zellen von dem Trägerelement lösen und somit einer weiteren Verwendung zur Verfügung stehen.

Vorzugsweise lösen sich die dentrischen Zellen spontan vom Trägerelement, wenn dieses durch entsprechende Modifizierung der Oberfläche vorbereitet ist. Es sind somit keine besonderen Maßnahmen für das Ernten der Zellen erforderlich.

Es kann jedoch auch vorteilhaft sein, wenn das Verfahren so ausgestaltet ist, dass sich die dendritischen Zellen durch mechanische Einwirkung vom Trägerelement lösen. Beispielsweise durch Schütteln oder Vibrieren kann somit der Zeitpunkt des Ablösens der dendritischen Zellen bestimmt werden.

Die Erfindung besteht ferner in einem Verfahren, welches sich dadurch auszeichnet, dass ein erfindungsgemäßer Bioreaktor verwendet wird. Bei einem solchen Verfahren werden die erfindungsgemäßen Vorteile des Bioreaktors umgesetzt.

Der Erfindung liegt die überraschende Erkenntnis zugrunde, dass organisches Material in einfacher und gut handhabbarer Weise gezüchtet werden kann, wenn ein Trägerelement geeignet bezüglich Membranen, Folien oder Geweben angeordnet wird. Durch die relative Anordnung kann einerseits eine geeignete Zuführung bzw. Abführung von Nährmedium erfolgen. Andererseits bieten die Membranen, Folien oder Gewebe die Möglichkeit der Isolierung des Trägerelementes und des darauf befindlichen organischen Materials nach außen. Es ist besonders vorteilhaft, dass insbesondere bei der beutelförmigen Ausführungsform eine sehr einfache Anordnung verwirklicht ist, so dass der Bioreaktor als Einmalartikel ausgelegt sein kann.

Die Erfindung wird nun mit Bezug auf die begleitenden Zeichnungen anhand von Ausführungsformen beispielhaft erläutert.
- Fig. 1: zeigt ein beutelartiges Modul in perspektivischer Darstellung;
- Fig. 2: zeigt eine seitliche Schnittansicht des beutelartigen Moduls gemäß Fig. 1;
- Fig. 3: zeigt eine weitere Ausführungsform eines beutelartigen Moduls in geschnittener Darstellung;
- Fig. 4: zeigt eine weitere Ausführungsform eines beutelartigen Moduls in geschnittener Darstellung, und
- Fig. 5: zeigt ein zylinderförmiges Modul.

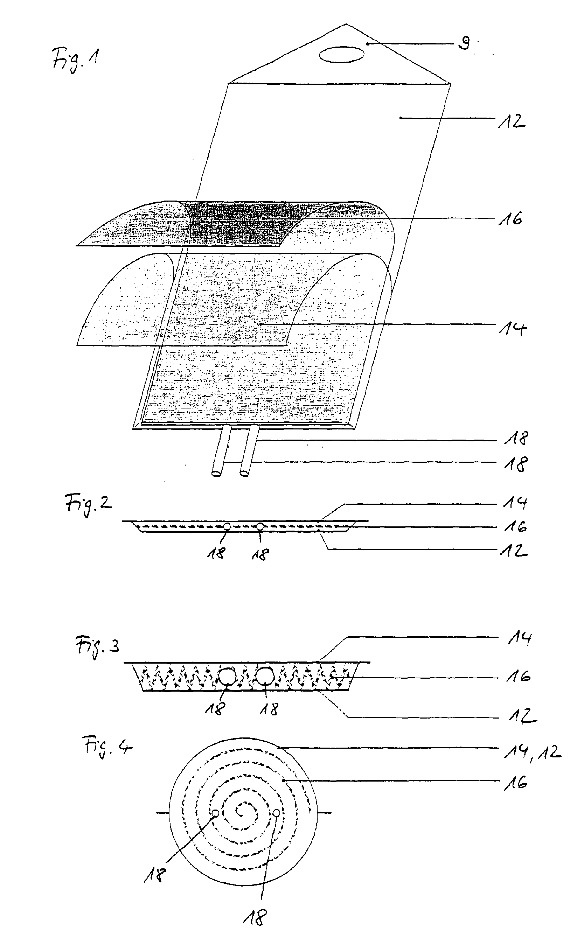

Fig. 1 zeigt den Aufbau eines erfindungsgemäßen Bioreaktors in Form eines Beutelmoduls. Auf einem ersten flächigen Element 12, welches mit einer Einprägung oder Vertiefung versehen ist, liegt ein Trägerelement 16, welches von einem zweiten flächigen Element 14 abgedeckt und umseitig verklebt oder verschweißt wird. Selbstverständlich können das erste 12 und das zweite flächige Element 14 einstückig ausgebildet sein. Es können Membrane, Folien oder Gewebe verwendet werden. Im fertigen Zustand bilden die flächigen Elemente 12, 14 einen geschlossenen Beutel mit einem definierten Aufnahmeraum und dem darin befindlichen Trägerelement 16. Es sind Rohrstücke als Zu- bzw. Abführungen 18 mit eingeschweißt, um Nährmedium oder Impfmedium bzw. die zu kultivierenden Zellen in das Beutelinnere zu leiten und aus diesem herauszuführen. Das erste flächige Element 12 weist an seiner Kopfseite eine Lasche 9 auf, welche zu Haltezwekken mit einer Öffnung versehen ist.

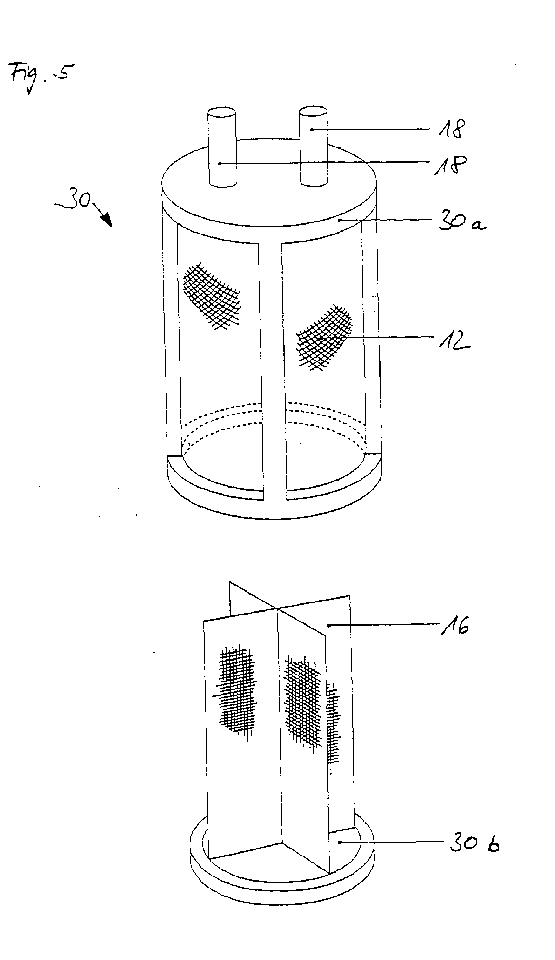

In Fig. 2 ist das Beutelmodul gemäß Fig. 1 in seitlich geschnittener Darstellung gezeigt. In dieser Darstellung ist ein flächiges Trägerelement 16 zwischen den flächigen Elementen 12, 14 angeordnet.

In Fig. 3 ist eine ähnliche Anordnung gezeigt. Allerdings hat das Trägerelement 16 eine gefaltete oder sogenannte plissierte Struktur.

In Fig. 4 bilden die flächigen Elemente 12, 14 zusammen einen Zylinder, und das Trägerelement 16 ist spiralförmig innerhalb des Zylinders angeordnet.

Fig. 5 zeigt gemäß dem zweiten Aspekt der Erfindung ein zylinderförmiges Modul mit Stützgerüst 30. In einen ersten Teil 30a des Stützgerüstes 30 ist ein äußeres flächiges Element 12 befestigt, beispielsweise durch Einkleben oder Einschweißen. Ein zweiter Teil 30b des Stützgerüstes ist als eine Bodenplatte ausgebildet, auf welcher das Trägerelement 16 in einer kreuzartigen Anordnung aufgebracht ist. Durch Verbinden der beiden Teile wird ein dichter Aufnahmeraum umschlossen.

Auf der Oberseite des ersten Teiles 30a des spritzgegossenen Gehäuses oder Stützgerüstes 30 sind Zu- bzw. Abführungen 18 mit ausgebildet, welche für den Transport von Nährmedium oder auch von einer Impfsubstanz verwendet werden können.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

## Patentansprüche

1. Bioreaktor zur Kultivierung organischen Materials mittels eines Nährmediums,
**dadurch gekennzeichnet**,
- dass ein Trägerelement (16) zum Tragen des organischen Materials vorgesehen ist, wobei das Trägerelement (16) für das Nährmedium zumindest teilweise durchlässig ist und
- dass das Trägerelement (16) zwischen zwei Abschnitten mindestens eines flächigen Elementes (12, 14) angeordnet und umschlossen ist, wobei eine beutelartige Struktur gebildet ist.

2. Bioreaktor nach Anspruch 1,
**dadurch gekennzeichnet**,
dass mindestens eine Zuführung (18) und eine Abführung (18) vorgesehen sind.

3. Bioreaktor nach einem der vorhergehenden Ansprüche,
**dadurch gekennzeichnet**,
dass das flächige Element (12, 14) eine Membran, eine Folie oder ein Gewebe ist.

4. Bioreaktor nach Anspruch 3,
**dadurch gekennzeichnet**,
dass die Membran oder die Folie ein Stützgewebe aufweist.

5. Bioreaktor nach einem der vorhergehenden Ansprüche,
**dadurch gekennzeichnet**,
dass das Trägerelement (16) zwischen dem flächigen Element (12, 14) eben angeordnet ist.

6. Bioreaktor nach einem der Ansprüche 1 bis 4,
**dadurch gekennzeichnet**,
dass das Trägerelement (16) zwischen dem flächigen Element (12, 14) wellenförmig angeordnet ist.

7. Bioreaktor nach einem der Ansprüche 1 bis 4,
**dadurch gekennzeichnet**,
dass das Trägerelement (16) zwischen dem flächigen Element (12, 14) wabenartig angeordnet ist.

8. Bioreaktor nach einem der Ansprüche 1 bis 4,
**dadurch gekennzeichnet**,
dass das Trägerelement (16) plissiert ist.

9. Bioreaktor nach einem der Ansprüche 1 bis 4,
**dadurch gekennzeichnet**,
dass das Trägerelement (16) gekreppt, gerollt, gefaltet oder geschichtet ist.

10. Bioreaktor nach einem der vorhergehenden Ansprüche,
**dadurch gekennzeichnet**,
dass das Trägerelement (16) ein Gewebe, insbesondere ein dreidimensional gewobenes Material ist.

11. Bioreaktor zur Kultivierung organischen Materials mittels eines Nährmediums,
**dadurch gekennzeichnet**,
- dass ein Trägerelement (16) zum Tragen des organischen Materials vorgesehen ist, wobei das Trägerelement (16) für das Nährmedium zumindest teilweise durchlässig ist,
- dass das Trägerelement (16) von einem Gehäuse umschlossen ist, welches ein Stützgerüst (30) umfasst, und
- dass an dem Stützgerüst (30) mindestens ein flächiges Element (12) angeordnet ist.

12. Bioreaktor nach Anspruch 11,
**dadurch gekennzeichnet**,
dass mindestens eine Zuführung (18) und mindestens eine Abführung (18) für das Nährmedium an dem Stützgerüst (30) vorgesehen sind.

13. Bioreaktor nach Anspruch 11 oder 12,
**dadurch gekennzeichnet**,
- dass das Stützgerüst (30) mindestens zwei Teile (30a, 30b) umfasst,
- dass an dem ersten Teil (30a) das flächige Element (12), insbesondere eine Membran, eine Folie oder ein Gewebe, angebracht ist, und
- dass an dem zweiten Teil (30b) das Trägerelement (16) angebracht ist.

14. Bioreaktor nach einem der Ansprüche 11 bis 13,
**dadurch gekennzeichnet**,
dass das Stützgerüst (30) als ein ein- oder mehrteiliges Spritzgussteil ausgebildet ist.

15. Verfahren zum Züchten organischen Materials, insbesondere dendritischer Zellen, bei dem
- ein Trägerelement (16) auf einen Abschnitt mindestens eines flächigen Elementes (12, 14), insbesondere einer Membran, einer Folie oder eines Gewebes, gelegt wird,
- das flächige Element (12, 14) zu einem Beutel zu-sammengeschweißt wird,
- das organische Material, insbesondere Monozyten und Nährmedium, in den Beutel eingebracht werden,
- der Beutel inkubiert wird, wobei organisches Material, insbesondere dendritische Zellen heranwachsen, und
- das organische Material, insbesondere die dentritischen Zellen vom Trägerelement (16) gelöst und geerntet werden.

16. Verfahren nach Anspruch 15,
**dadurch gekennzeichnet**,
dass sich das organische Material oder die dendritischen Zellen spontan vom Trägerelement (16) lösen.

17. Verfahren nach Anspruch 15 oder 16,
**dadurch gekennzeichnet**,
dass das organische Material oder die dendritischen Zellen durch mechanische Einwirkung vom Trägerelement (16) gelöst werden.

18. Verfahren nach einem der Ansprüche 15 bis 17,
**dadurch gekennzeichnet**,
dass eine Vorrichtung gemäß einem der Ansprüche 1 bis 10 verwendet wird.
